# EUROPÄISCHE PATENTSCHRIFT

(11) **EP 2 037 891 B1**
(45) Veröffentlichungstag und Bekanntmachung des Hinweises auf die Patenterteilung: **24.10.2012**
(21) Anmeldenummer: 07764644.6
(22) Anmeldetag: 14.06.2007
(51) Int. Cl.: A61K 9/20, A61K 31/4184, A61K 47/14, A61K 47/34, A61K 31/54, C07D 413/10, A61K 9/50

(54) **TABLETTE ENTHALTEND CANDESARTAN CILEXETIL**
TABLETS COMPRISING CANDESARTAN CILEXETIL
COMPRIME RENFERMANT DU CANDESARTAN CILEXETIL

(30) Priorität: 20.06.2006 CH 998062006
(43) Veröffentlichungstag der Anmeldung: 25.03.2009
(73) Patentinhaber: Siegfried International AG, 4800 Zofingen (CH)
(72) Erfinder: RÖHRICH, Tillmann, 4310 Rheinfelden (CH); BUEB, Waltraud, 4663 Aarburg (CH); SCHWEINBERGER, Enno, 4800 Zofingen (CH)
(74) Vertreter: Braun, André jr.
(86) Internationale Anmeldenummer: PCT/EP2007/005225
(87) Internationale Veröffentlichungsnummer: WO 2007/147514

(56) Entgegenhaltungen:
- WO-A-2006/079496
- WO-A1-2005/084648
- WO-A2-2005/079751
- US-A1- 2004 005 358

## Beschreibung

Die vorliegende Erfindung betrifft eine feste pharmazeutisch wirksame Zusammensetzung in Form von Tabletten enthaltend Candesartan Cilexetil, für die orale Verabreichung.

Die Verbindung Candesartan Cilexetil entspricht der chemischen Formel

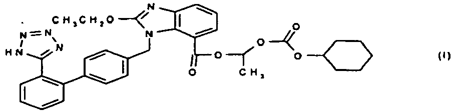

Candesartan Cilexetil wird chemisch als (±)-1-[[(Cyclohexyloxy)carbonyl]oxy]ethyl-2-ethoxy-1-[[2'-(1H-tetrazol-5-yl)-[1,1'-biphenyl]-4-yl]methyl]-1H-benzimidazole-7-carboxylate bezeichnet. Candesartan Cilexetil ist ein Angiotensin II Rezeptor Antagonist. Candesartan Cilexetil wird als Vorstufe von Candesartan verwendet, wobei diese nach der Einnahme im Körper zu Candesartan hydrolysiert wird.

Candesartan Cilexetil als Wirkstoff in Tablettenform ist vergleichsweise instabil und bildet, insbesondere bei der Langzeitlagerung, unerwünschte Nebenprodukte. So bilden sich beispielsweise Desethyl-Candesartan und verwandte Verbindungen, welche den Gehalt an Wirkstoff in der Tablette reduzieren. Diese Zersetzung kann oft auch den in der Tablette verwendeten Zusatzstoffen zugeschrieben werden, doch ist dies schwierig bestimmbar.

Die internationale Patentveröffentlichungen WO 2005/079751 sowie WO 2005/084648 beschäftigen sich mit der Stabilisierung von Candesartan Cilexetil in festen Zubereitungen in Gegenwart anderer Inhaltsstoffe zur verbesserten Behandlung von Bluthochdruck. Bei Dokumente offenbaren den Wirkstoff Candesartan Cilexetil welcher in Tablettenform oder aber auch Kapseln mit verschiedenen filmbildenden Materialien überzogen ist wie z.B. gewisse Fettsäureester und gewisse wasserlösliche Polymere zur Erhöhung der Stabilität.

Die Publikation US 2004/005358 offenbart eine Liste von verschiedenen Angiotensin II Receptorantagonist wie z.B. Candesartan Cilextil mit Vasopeptidase Inhibitor, die in umhüllten Granulaten oder Tabletten vorliegen. Das Überzugsmaterial kann aus einer Liste gewählt werden und u.a. auch Triethylcitrate, Diethylphthalate oder Dibutylsebacate sein.

Es wurde nun gefunden, dass Candesartan Cilexetil mit üblichen Zusatzstoffen zu festen stabilen Zusammensetzungen in Form von Tabletten, verarbeitet bzw. verpresst werden kann, wenn die Zusammensetzung Candesartan Cilexetil und gegebenenfalls weitere Wirkstoffe, sowie übliche Zusatzstoffe enthält, und in dieser Zusammensetzung die Oberfläche mindestens des Wirkstoffs Candesartan Cilexetil mit einem Überzug, bestehend aus einer Verbindung oder einem Gemisch von Verbindungen, ausgewählt aus Tri(C₁-C₆)alkylcitrat, Di(C₁-C₆)alkylphthalat, Di(C₁-C₆)alkylsebacat und Polydimethylsiloxanen, versehen ist, wobei diese Verbindung oder das Gemisch dieser Verbindungen alleine den Überzug bildet oder mindestens 40 Gew.-% des Gesamtgewichts des Überzugs darstellt.

Erfindungswesentlich ist, dass die Oberfläche des Wirkstoffs Candesartan Cilexetil vor der Verpressung mit dem erfindungsgemässen Überzug versehen wird. Dabei kann der Wirkstoff alleine, oder im Gemisch mit allfälligen Zusatzstoffen, mit dem erfindungsgemässen Überzug versehen sein. Der Wirkstoff und allenfalls anwesende weitere Wirkstoffe liegen vorgängig zur Beschichtung mit dem Überzug vorzugsweise in feinkörniger Form vor. Die Zusammensetzung kann gegebenenfalls vorgängig zur Verpressung granuliert werden.

Für die Beschichtung verwendet man vorzugsweise eine Verbindung, welche ausgewählt ist aus Tri (C₁-C₆) alkylcitrat, Di (C₁-C₆)-alkylphthalat, Di(C₁-C₆)alkylsebacat und Polydimethylsiloxanen mit einer Viskosität im Bereich von 20-1300 cSt, oder ein Gemisch dieser Verbindungen. Überraschenderweise stabilisiert diese Verbindung oder ein Gemisch dieser Verbindungen den Wirkstoff sowohl in der Ausgangsmischung als auch in der Tablette, so dass kaum Zerfallserscheinungen, auch nach längerer Lagerzeit, auftreten.

Die vorliegende Erfindung ist in den Patentansprüchen definiert. Insbesondere betrifft die Erfindung eine pharmazeutisch wirksame Zusammensetzung in Form einer Tablette, welche Candesartan Cilexetil und gegebenenfalls weitere Wirkstoffe, sowie übliche Zusatzstoffe enthält, dadurch gekennzeichnet, dass in dieser Zusammensetzung die Oberfläche mindestens des Wirkstoffs Candesartan Cilexetil mit einem Überzug, bestehend aus einer Verbindung oder einem Gemisch von Verbindungen, ausgewählt aus Tri(C₁-C₆)alkylcitrat, Di(C₁-C₆)alkylphthalat, Di (C₁-C₆) alkylsebacat und Polydimethylsiloxanen, versehen ist, wobei diese Verbindung oder das Gemisch dieser Verbindungen alleine den Überzug bildet oder einen wesentlichen Bestandteil des Überzugs darstellt.

Die vorliegende Erfindung betrifft auch die Ausgangsmischung, enthaltend den Wirkstoff Candesartan Cilexetil, gegebenenfalls weitere Wirkstoffe, sowie übliche Zusatzstoffe, dadurch gekennzeichnet, dass in dieser Ausgangsmischung die Oberfläche mindestens des Wirkstoffs Candesartan Cilexetil mit dem erfindungsgemässen Überzug versehen ist.

Die Ausgangsmischung kann vorgängig zur Verpressung granuliert werden. In diesem Sinne betrifft die Erfindung auch ein Granulat, welches geeignet ist für die Herstellung der erfindungsgemässen Tablette, dadurch gekennzeichnet, dass dieses Granulat durch Granulation der erfindungsgemässen Ausgangsmischung hergestellt wurde. Die Ausgangsmischung liegt vor der Verpressung vorzugsweise in feinkörniger Form vor.

In diesem Sinne betrifft die vorliegende Erfindung eine Tablette, welche die erfindungsgemässe Ausgangsmischung und/oder das erfindungsgemässe Granulat in verpresster Form enthält, worin die Oberfläche mindestens des Wirkstoffs Candesartan Cilexetil mit einem erfindungsgemässen Überzug versehen ist.

Die vorliegende Erfindung betrifft auch ein Verfahren zur Herstellung der erfindungsgemässen Tablette.

Die vorliegende Erfindung betrifft auch die Verwendung der erfindungsgemässen Zusammensetzung, bzw. Tablette für die orale Verabreichung, als Angiotensin II Rezeptor Antagonist, insbesondere zur Behandlung von Bluthochdruck.

Von Candesartan Cilexetil sind unterschiedliche polymorphe Strukturen bekannt. Die vorliegende Erfindung ist für alle diese Strukturen anwendbar.

Candesartan Cilexetil sowie allenfalls anwesende weitere Wirkstoffe liegen in der Tablette vorzugsweise in fein verteilter Form vor, vorzugsweise in einer Korngrössenverteilung, bei welcher etwa 90% der Körner einen mittleren Durchmesser von weniger als 20 Micron (D₉₀<20µm) und vorzugsweise etwa 50% der Körner einen mittleren Durchmesser von weniger als 10 Micron (D₅₀<10µm) aufweisen.

Die erfindungsgemässe Zusammensetzung enthält die stabilisierend wirkende Verbindung oder das Gemisch der stabilisierend wirkenden Verbindungen vorzugsweise in einer Konzentration im Bereich von etwa 2.0 Gew.-% bis etwa 45 Gew.-%, vorzugsweise im Bereich von etwa 5 Gew.-% bis etwa 40 Gew.-%, bezogen auf das Gewicht des anwesenden Candesartan Cilexetils. Die Konzentrationen sind in der Regel nicht kritisch und können vom Fachmann fallweise optimiert werden. So genügen bereits 0.80 mg Triethylcitrat für eine Tablette, welche 16 mg Wirkstoff enthält.

Die erfindungsgemässe Zusammensetzung bzw. die Tablette enthält die stabilisierend wirkende Verbindung oder das Gemisch der stabilisierend wirkenden Verbindungen vorzugsweise in einer Konzentration im Bereich von etwa 0.2 Gew.-% bis etwa 5.0 Gew.-%, vorzugsweise etwa 0.5 Gew.-%, bezogen auf das Gewicht der Zusammensetzung bzw. auf das Tablettengewicht.

Als "weitere Wirkstoffe" kommt insbesondere Hydrochlorothiazid (CAS Nr. [58-93-5]) in Frage, wobei das Gewichtsverhältnis von Candesartan Cilexetils zu Hydrochlorthiazid vorzugsweise im Bereich von 3 : 1 bis 1 : 3, vorzugsweise im Bereich von 2 : 1 bis 1 : 2, und insbesondere bei etwa 1.3 : 1 bis 1 : 1.6 liegt. Hydrochlorothiazid wirkt als Diuretikum. Auch andere Diuretika kommen als weitere Wirkstoffe in Frage.

In der Ausgangsmischung ist die Oberfläche mindestens des Wirkstoffs Candesartan Cilexetil mit einem Überzug, bestehend aus einer Verbindung oder einem Gemisch von Verbindungen, ausgewählt aus Tri(C₁-C₆)alkylcitrat, Di(C₁-C₆)alkylphthalat, Di(C₁-C₆)alkylsebacat und Polydimethylsiloxanen, versehen ist, wobei diese Verbindung oder das Gemisch dieser Verbindungen alleine den Überzug bildet oder mindestens 40 Gew.-%, vorzugsweise mindestens 50 Gew.-%, vorzugsweise mindestens 80 Gew.-% und vorzugsweise mindestens 90 Gew.-% des Gesamtgewichts des Überzugs darstellt.

Tri(C₁-C₆)alkylcitrate sind beispielsweise Trimethylcitrat, Triethylcitrat, Tripropylcitrat, Tributylcitrat, vorzugsweise Triethylcitrat, Tripropylcitrat, Tributylcitrat, vorzugsweise Triethylcitrat und Tributylcitrat, vorzugsweise Triethylcitrat.

Di(C₁-C₆)alkylphthalate sind beispielsweise Dimethylphthalat, Diethylphthalat, Dipropylphthalat, Dibutylphthalat, vorzugsweise Dimethylphthalat, Diethylphthalat und Dibutylphthalat, vorzugsweise Dimethylphthalat und Diethylphthalat.

Di(C₁-C₆)alkylsebacate sind beispielsweise Dimethylsebacat, Diethylsebacat, Dipropylsebacat, Dibutylsebacat, vorzugsweise Dimethylsebacat, Diethylsebacat, Dibutylsebacat, vorzugsweise Diethylsebacat und Dibutylsebacat, vorzugsweise Dibutylsebacat.

Vorzugsweise ist das Polydimethylsiloxan eine flüssige Verbindung mit einer Viskosität vorzugsweise im Bereich von 20-1300 cSt. Vorzugsweise ist das Polydimethylsiloxan ein lineares Polydimethylsiloxan mit einem Polymerisationsgrad (n) im Bereich von n = 20-400.

Candesartan Cilexetil ist in der Zusammensetzung pro Tablette in einer Menge von 2 mg bis 50 mg anwesend, beispielsweise in einer Menge von 2 mg, 4 mg, 8 mg, 10 mg, 16 mg, 24 mg, 32 mg oder 45 mg. Der Anteil des Wirkstoffs am Gesamtgewicht der Tablette beträgt etwa 1 Gew.-% bis etwa 20 Gew.%.

Als pharmazeutisch verwendbare "übliche Zusatzstoffe" (Excipients) kann die erfindungsgemässe Zusammensetzung insbesondere Füllstoffe, Bindemittel, Schmiermittel, Disintegrants (Sprengmittel), Farbstoffe und Geschmackstoffe enthalten. Die Zusatzstoffe ergänzen das Gesamtgewicht der Zusammensetzung auf 100 Gew.-%. Die qualitative und quantitative Eignung und Verwendung dieser Hilfsstoffe ist dem Fachmann bekannt, wobei das Verhältnis der einzelnen Zusatzstoffe untereinander vom Fachmann in an sich bekannter Weise optimiert werden kann.

Füllstoffe sind beispielsweise Stärken, insbesondere pregelatinisierte Stärken, Maisstärke, Cellulosen, Lactose Monohydrat, Zucker, Sucrose, Glucose, Fructose, Sorbitol, microcrystalline Cellulose, Dextrin, Dextrose, Mannitol, Sorbitol, Calciumsalze, wie Calciumkarbonat, Calciumsulfat, Kaolin, sowie Mischungen derselben.

Bindemittel sind beispielsweise Methylcellulose, Hydroxypropylcellulose (HPC), Polyvinylpyrrolidon, Gelatine, Gummi Arabicum, Ethylcellulose, Polyvinylalkohol, Tragacanth, Natriumalginat, Propylenglycole und Mischungen dieser Verbindungen.

Schmiermittel sind beispielsweise Magnesiumstearate, kolloidales Silika, Calciumstearat, Talk, hydriertes Castoröl, microcrystallines Wachs, Bienenwachs, Polyethylenglycol, Natrium stearylfumarat und Mischungen dieser Verbindungen. Diese wirken oft auch als Entformungsmittel. Pharmazeutisch zugelassene Farbstoffe und Geschmackstoffe sind bekannt und werden in den an sich bekannten Mengen eingesetzt.

Disintegrants (Sprengmittel) sind beispielsweise Calcium Carboxymethylcellulose, kolloidales Siliziumdioxid, Stärke, Natrium Croscarmellose, Crospovidon, Natrium Stärkeglycolat und Mischungen dieser Verbindungen.

Eine mögliche Ausführungsform für ein Verfahren zur Herstellung der erfindungsgemässen Zusammensetzung ist dadurch gekennzeichnet, dass man Candesartan Cilexetil, mit mindestens einer stabilisierend wirkenden Verbindung überzieht, und gegebenenfalls mit einem oder mehreren weiteren Wirkstoffen, sowie mit mindestens einem Zusatzstoff, vorzugsweise mit mindestens einem Füllstoff und mindestens einem Bindemittel, vorzugsweise auch mit mindestens einem Schmiermittel und einem Sprengmittel, intensiv mischt und das erhaltene Gemisch zu einer Tablette verpresst. Dabei können auch sämtliche Zusatzstoffe mit einer stabilisierend wirkenden Verbindung überzogen sein.

Eine weitere Ausführungsform des erfindungsgemässen Verfahrens besteht darin, dass man Candesartan Cilexetil, mit mindestens einer stabilisierend wirkenden Verbindung intensiv mischt oder überzieht, und gegebenenfalls mit einem oder mehreren weiteren Wirkstoffen, sowie mit mindestens einem Zusatzstoff, vorzugsweise mit mindestens einem Füllstoff und mindestens einem Bindemittel mischt, das Gemisch in Gegenwart von Wasser granuliert, das erhaltene Granulat trocknet und das trockene Granulat vorzugsweise mit mindestens einem Schmiermittel und mit mindestens einem Sprengmittel mischt und zu einer Tablette verpresst. Dabei können sämtliche Zusatzstoffe mit einer stabilisierend wirkenden Verbindung überzogen sein. Zur Granulation verwendet man vorzugsweise die an sich bekannte Feuchtgranulierung, wobei man dieses Verfahren in einem an sich bekannten Granulator durchführt.

Ein weiteres Verfahren zur Herstellung der erfindungsgemässen Zusammensetzung besteht darin, dass man in einem Fliessbettverfahren (i) Candesartan Cilexetil, gegebenenfalls im Gemisch mit einem weiteren Wirkstoff (z.B. Hydrochlorothiazid) oder weiteren Wirkstoffen, zusammen mit mindestens einem Füllmittel und ggf. einem Sprengmittel (z.B. Lactose Monohydrat und/oder Maisstärke) fluidisiert, und (ii) diesem fluidisierten Gemisch eine stabilisierend wirkende Verbindung gelöst oder emulgiert in Wasser (z.B. Triethylcitrat in Wasser) zusetzt, wobei das anwesende Gemisch mit der stabilisierend wirkenden Verbindung überzogen wird, gegebenenfalls (iii) das Gemisch im Fliessbett mit Bindemittellösung (z.B. Hyprolose) nach bekannter Weise granuliert, das erhaltene Gemisch (als Pulver oder Granulat) trocknet, das trockene Gemisch dem Fliessbett entnimmt, siebt und desagglomeriert, (iv) dem Gemisch ein Sprengmittel (z.B. Ac-Di-Sol) sowie ein Schmiermittel (z.B. Magnesiumstearat) zusetzt und nach gleichmässiger Verteilung der Komponenten das erhaltene Gemisch zu Tabletten verpresst.

Ein weiteres Verfahren zur Herstellung der erfindungsgemässen Zusammensetzung besteht darin, dass man (i) Candesartan Cilexetil, gegebenenfalls im Gemisch mit einem weiteren Wirkstoff (z.B. Hydrochlorothiazid) oder weiteren Wirkstoffen, zusammen mit mindestens einer stabilisierend wirkenden Verbindung in Wasser suspendiert und (ii) mit oder ohne Bindemittel (z.B. Hydroxypropylcellulose) in einem Fliessbettverfahren auf einen oder mehrere Trägerstoffe/Füllmittel (z.B. Lactose Monohydrat und/oder Maisstärke) aufsprüht; das Gemisch im Fliessbett wird gegebenenfalls granuliert; das erhaltene Gemisch (als Pulver oder Granulat) wird dann getrocknet (iii). Das trockene Gemisch wird dem Fliessbett entnommen, gesiebt und desagglomeriert, (iv) dem Gemisch wird ein Sprengmittel (z.B. Ac-Di-Sol) sowie ein Schmiermittel (z.B. Magnesiumstearat) zugesetzt und nach gleichmässiger Verteilung der Komponenten wird das erhaltene Gemisch zu Tabletten verpresst.

Für die Tablettierung des Gemisches verwendet man eine übliche Tablettiermaschine, wobei für die Tablettierung Drucke im Bereich von 2 KN bis 30KN, vorzugsweise 5KN bis 15 KN zur Anwendung kommen.

Die derart erhaltene verpresste Mischung bzw. Tablette kann als Tablettenkern mit einem Überzug versehen werden. Für die Herstellung des Überzugs verwendet man vorzugsweise an sich bekannte kommerziell verfügbare Verbindungen, wie beispielsweise Ethylcellulose, Hydroxypropylmethylcellulose (HPMC), Hydroxypropylcellulose, Methylcellulose, Carboxymethylcellulose (CMC), Hydroxymethylcellulose HMC), Hydroxyethylcellulose, Hydroxypropylmethylphthalat (HPMP), Celluloseacetat, sowie an sich bekannten Polyethylenglycole und Polymere und Copolymere von Methacrylsäure. Diese werden in an sich bekannter Weise aus Lösungsmitteln, wie beispielsweise Wasser oder Methanol, Ethanol, Isopropylalkohol, Aceton, gegebenenfalls im Gemisch mit Wasser auf die Pressmischung bzw. den Tablettenkern aufgebracht. Die folgenden Beispiele erläutern die Erfindung.

### Beispiel 1 (Herstellung einer Tablette)

Die in den Beispielen verwendeten Zusatzstoffe haben die folgenden Funktionen:

| Zusatzstoff: | Funktion: | | Zusatzstoff: | Funktion: |
|---|---|---|---|---|
| Triethylcitrat, Simethicon | stabilisierend wirkende Verbindung | | Hydroxypropyl cellulose (Hyprolose) | Bindemittel |
| Lactose Monohydrat | Füllstoff | | Croscarmellose | Disintegrant (Sprengmittel) |
| Maistärke | Füllstoff/ Disintegrant | | Magnesiumstearat | Schmiermittel/ Formentrennmittel |

Candesartan Cilexil, Lactose Monohydrat (wasserlöslich, z.B. Pharmatose® von der Firma DMV) und Maisstärke wurden mit den in Tabelle 1 angegebenen Mengen in einer Wirbelschichtapparatur (GPCG 3.1. der Firma Glatt) fluidisiert. Eine wässerige Emulsion der stabilisierend wirkenden Verbindung [(a) Triethylcitrat; (b) Simethicon] wurde in die Wirbelschicht eingespeist, so dass die Pulvermischung mit einer Schicht der stabilisierend wirkenden Verbindung überzogen wurde. Hierauf wurde eine wässerige Lösung von Hydroxypropylcellulose (HPC, Klucel®) gelöst in 9 Teilen Wasser eingespeist, so dass ein Granulat erhalten wurde. Das Granulat wurde anschliessend in der Wirbelschicht bei 60°C Zuluft, bis zur Gleichgewichtsfeuchte getrocknet, gesiebt und dann mit Calcium Carmellose und Magnesiumstearat gemischt und zu Tabletten verpresst.

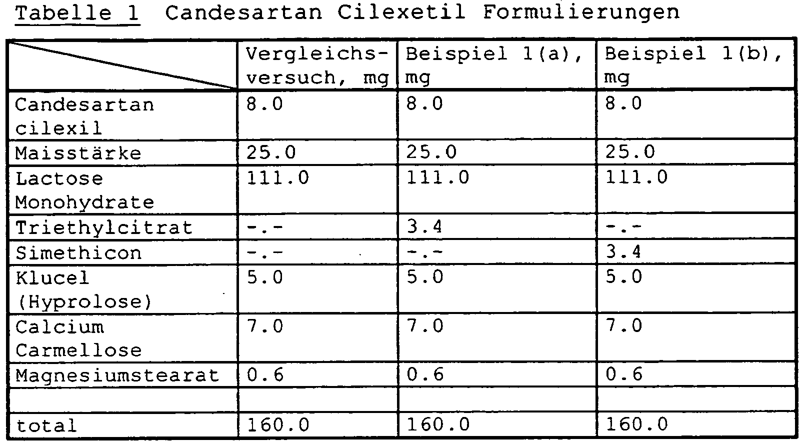

### Beispiele 2-5

Beispiel 1 wurde wiederholt, jedoch mit den in Tabelle 2 angegebenen Verbindungen und Konzentrationen. Triehylcitrat wurde auf ein Gemisch des Wirkstoffs, Lactose und Maisstärke aufgebracht, mit einer wässerigen Lösung von Hydroxypropylcellulose granuliert, mit den in Tabelle 2 aufgelisteten Zusatzstoffen zur fertigen Endmischung aufgearbeitet und mit einer Rundläufertablettenpresse (Fette P1) zu Tabletten verpresst. Tabletten mit zwei unterschiedlichen Pressdrucken, bzw. zwei unterschiedlichen Härten (50N und 80N) wurden hergestellt und in offenen braunen Glasbehältern während mindestens zwei Wochen bei 50°C gelagert. Gleichzeitig wurde die nicht druckbelastete Endmischung als Referenzmuster zum Vergleich während derselben Zeitspanne bei 4°C in geschlossenen braunen Glasbehältern gelagert.

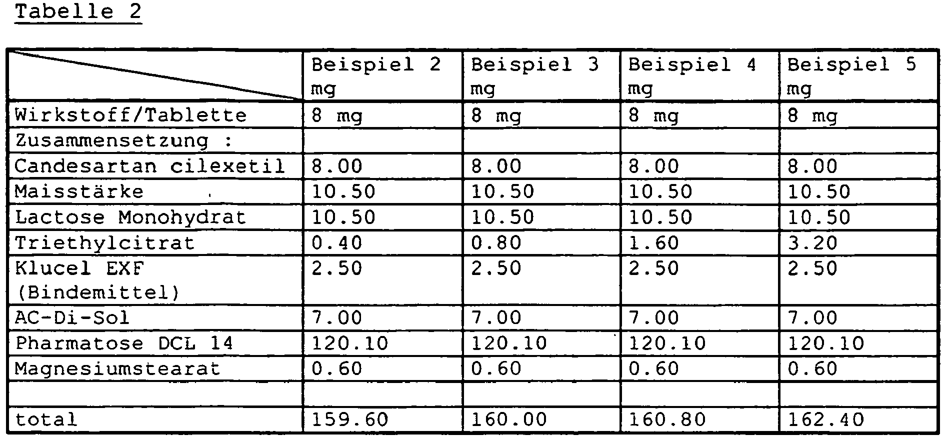

Pharmatose DCL 14 = Lactose monohydrate, sprühgetrocknet für Direkttablettierung

### Beispiele 6-12

Die Beispiele 1-5 wurden wiederholt, jedoch mit der Massnahme, dass die in Tabelle 3 angegebenen Zusammensetzungen verwendet wurden.

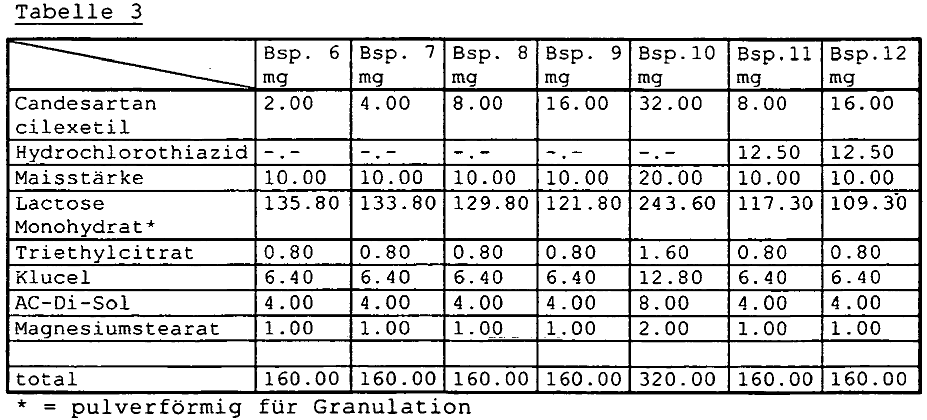

### Testresultate aus Beispiel 1

Die in Beispiel 1 hergestellten Tabletten wurden in offenen Flaschen während zwei Wochen bei 50°C gelagert. Dann wurde von den Tabletten das Verunreinigungsprofil bestimmt. Ein Vergleichsmuster, gelagert in einer verschlossenen Flasche während derselben Zeit, jedoch bei 4°C, wurde als Referenz verwendet. Das Verunreinigungsprofil ist charakterisiert durch Desethyl-Candesartan Cilexetil als hauptsächliche Verunreinigung, welche eine deutliche Zunahme während der Lagerung zeigt, nämlich bei RT [min] 8.2. Der Haupt-Peak von Candesartan Cilexetil ist bei RT [min] 16.5. Die Resultate sind in Tabelle 4 aufgelistet.

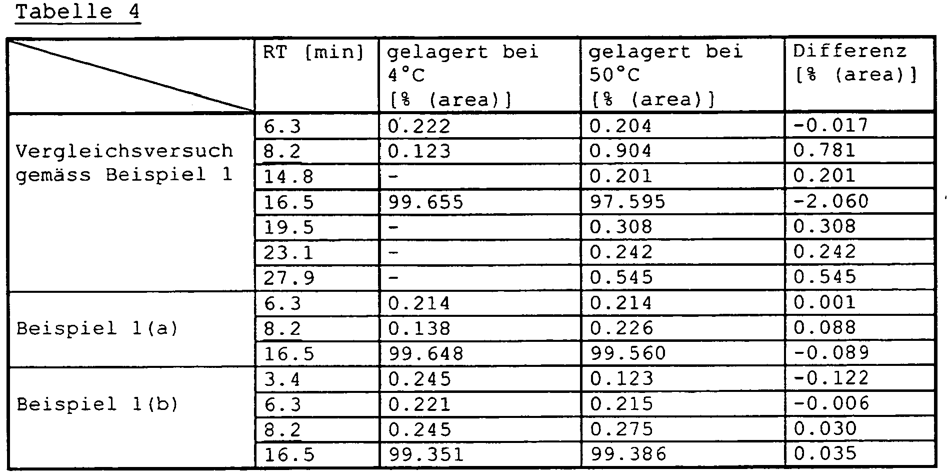

Die Verunreinigung bei RT = 6.3 ist ein Nebenprodukt in Verbindung mit der Synthese. Die Menge bleibt konstant während der Lagerung und beeinflusst die Stabilität und das Ergebnis nicht.

Eine kritische Verbindung im Stabilitätstest ist die Zunahme von Desethyl Candesartan Cilexetil (RT = 8.2), welches ein Abbauprodukt von Candesartan Cilexetil darstellt. Die Verwendung von Triethylcitrate und Dimethicon (CAS Nr. [9006-65-9]) beweisen die stabilisierende Wirkung dieser Verbindung für Candesartan Cilexetil Tabletten während der Lagerung.

### Testresultate aus den Beispielen 2 bis 5

Die Tabletten aus den Beispielen 2, 3, 4 und 5 wurden mit einer geänderten HPLC-Methode untersucht, was zu anderen Retentionszeiten führte. Die erhaltenen Resultate sind in Tabelle 5 angegeben. Dabei entsprechen die Peaks den folgenden Verbindungen:
Peak at 1.7 - 1.8 min = Desethyl-Candesartan Cilexetil
Peak at 3.8 - 3.9 min = Candesartan Cilexetil
Peak at 9.1 - 9.2 min = N-Ethyl Candesartan Cilexetil

**Tabelle 5**

| | RT[min] | Gelagert bei 50°C [%(area)] nach 2 Wochen | Differenz (50°C-4°C) [%(area)] nach 2 Wochen |
|---|---|---|---|
| Beispiel 2 | 1.8 | 0.140 | 0.092 |
| | 3.9 | 99.44 | -0.12 |
| | 9.1 | 0.069 | 0.029 |
| Beispiel 3 | 1.8 | 0.078 | 0.035 |
| | 3.9 | 99.24 | -0.33 |
| | 9.1 | 0.053 | 0.011 |
| Beispiel 4 | 1.8 | 0.077 | 0.031 |
| | 3.9 | 99.42 | -0.14 |
| | 9.1 | 0.055 | 0.016 |
| Beispiel 5 | 1.8 | 0.079 | 0.035 |
| | 3.9 | 99.51 | -0.08 |
| | 9.1 | 0.053 | 0.022 |

Die Testresultate zeigen deutlich die stabilisierende Wirkung von Triethylcitrat.

### Testresultate mit weiteren stabilisierend wirkenden Verbindungen

Analoge Resultate werden erhalten wenn man, analog zu den vorgehenden Beispielen, die folgenden stabilisierend wirkenden Verbindungen anstelle von Triethylcitrat und Simethicon verwendet: Trimethylcitrat, Tributylcitrat, Dibutylsebacat, Dimethylphthalat, Diethylphthalat und/oder Dibutylphthalat.

## Patentansprüche

1. Zusammensetzung in Form einer Tablette oder eines Granulats, welche Candesartan Cilexetil und gegebenenfalls weitere Wirkstoffe, sowie übliche Zusatzstoffe enthält, **dadurch gekennzeichnet, dass** in dieser Zusammensetzung die Oberfläche mindestens des Wirkstoffs Candesartan Cilexetil mit einem Überzug, bestehend aus einer Verbindung oder einem Gemisch von Verbindungen, ausgewählt aus Tri (C₁-C₆) alkylcitrat, Di (C₁-C₆) alkylphthalat, Di (C₁-C₆) alkylsebacat und Polydimethylsiloxanen, versehen ist, wobei diese Verbindung oder das Gemisch dieser Verbindungen alleine den Überzug bildet oder mindestens 40 Gew.-% des Gesamtgewichts des Überzugs darstellt.

2. Zusammensetzung nach Anspruch 1, **dadurch gekennzeichnet, dass** Candesartan Cilexetil sowie allenfalls anwesende weitere Wirkstoffe in fein verteilter Form vorliegen, vorzugsweise mit einer Korngrössenverteilung, bei welcher 90% der Körner einen mittleren Durchmesser von weniger als 20 Micron (D₉₀<20µm) und vorzugsweise 50% der Körner einen mittleren Durchmesser von weniger als 10 Micron (D₅₀<10µm) aufweisen.

3. Zusammensetzung nach Anspruch 1 oder 2, **dadurch gekennzeichnet, dass** die stabilisierend wirkende Verbindung oder das Gemisch der stabilisierend wirkenden Verbindungen in einer Konzentration im Bereich von 2.0 Gew.-% bis etwa 45 Gew.-%, vorzugsweise im Bereich von 5 Gew.-% bis 40 Gew.-%, bezogen auf das Gewicht des anwesenden Candesartan Cilexetils, vorliegen.

4. Zusammensetzung nach einem der Ansprüche 1-3, **dadurch gekennzeichnet, dass** die stabilisierend wirkende Verbindung oder das Gemisch der stabilisierend wirkenden Verbindungen mindestens 50 Gew.-%, vorzugsweise mindestens 80 Gew.-% und vorzugsweise mindestens 90 Gew.-% des Gesamtgewichts des Überzugs bildet.

5. Zusammensetzung nach einem der Ansprüche 1-4, **dadurch gekennzeichnet, dass** die stabilisierend wirkende Verbindung oder das Gemisch der stabilisierend wirkenden Verbindungen in einer Konzentration im Bereich von 0.2 Gew.-% bis 5.0 Gew.-%, vorzugsweise etwa 0.5 Gew.-%, bezogen auf das Gewicht der Zusammensetzung, vorliegt.

6. Zusammensetzung nach einem der Ansprüche 1-5, **dadurch gekennzeichnet, dass** diese als weiteren Wirkstoff Hydrochlorothiazid (CAS Nr. [58-93-5]) oder ein anderes Diuretikum enthält, wobei das Gewichtsverhältnis von Candesartan Cilexetils zu Hydrochlorthiazid vorzugsweise im Bereich von 3:1 bis 1:3, vorzugsweise im Bereich von 2:1 bis 1:2, und insbesondere bei etwa 1.3:1 bis 1:1.6 liegt.

7. Zusammensetzung nach einem der Ansprüche 1-6, **dadurch gekennzeichnet, dass** das Tri(C₁-C₆)alkylcitrat ausgewählt ist aus Trimethylcitrat, Triethylcitrat, Tripropylcitrat, Tributylcitrat, vorzugsweise aus Triethylcitrat, Tripropylcitrat, Tributylcitrat, vorzugsweise aus Triethylcitrat und Tributylcitrat, und vorzugsweise Triethylcitrat darstellt.

8. Zusammensetzung nach einem der Ansprüche 1-6, **dadurch gekennzeichnet, dass** das Di(C₁-C₆)alkylphthalat ausgewählt ist aus Dimethylphthalat, Diethylphthalat, Dipropylphthalat, Dibutylphthalat, vorzugsweise aus Dimethylphthalat, Diethylphthalat und Dibutylphthalat, und vorzugsweise Dimethylphthalat und/oder Diethylphthalat darstellt.

9. Zusammensetzung nach einem der Ansprüche 1-6, **dadurch gekennzeichnet, dass** das Di(C₁-C₆)alkylsebacate ausgewählt ist aus Dimethylsebacat, Diethylsebacat, Dipropylsebacat, Dibutylsebacat, vorzugsweise aus Dimethylsebacat, Diethylsebacat, Dibutylsebacat, vorzugsweise aus Diethylsebacat und Dibutylsebacat, und vorzugsweise Dibutylsebacat darstellt.

10. Zusammensetzung nach einem der Ansprüche 1-6, **dadurch gekennzeichnet, dass** das Polydimethylsiloxan eine flüssige Verbindung mit einer Viskosität vorzugsweise im Bereich von 20-1300 cSt, und vorzugsweise ein lineares Polydimethylsiloxan mit einem Polymerisationsgrad (n) im Bereich von n = 20-400, darstellt.

11. Zusammensetzung nach einem der Ansprüche 1-10, **dadurch gekennzeichnet, dass** Candesartan Cilexetil in der Zusammensetzung pro Tablette in einer Menge von 2 mg bis 50 mg anwesend ist, vorzugsweise in einer Menge von 2 mg, 4 mg, 8 mg, 10 mg, 16 mg, 24 mg, 32 mg oder 45 mg und der Anteil des Wirkstoffs am Gesamtgewicht der Tablette 1 Gew.-% bis etwa 20 Gew.-% beträgt.

12. Zusammensetzung nach einem der Ansprüche 1-11, **dadurch gekennzeichnet, dass** diese als pharmazeutisch verwendbare übliche Zusatzstoffe Füllstoffe, Bindemittel, Schmiermittel, Sprengmittel, Farbstoffe und/oder Geschmackstoffe enthält.

13. Verfahren zur Herstellung einer Tablette nach einem der Ansprüche 1-12, **dadurch gekennzeichnet, dass** man die Oberfläche mindestens des Wirkstoffs Candesartan Cilexetil mit einem Überzug, bestehend aus einer Verbindung oder einem Gemisch von Verbindungen, ausgewählt aus Tri (C₁-C₆) alkylcitrat, Di (C₁-C₆)-alkylphthalat, Di(C₁-C₆)alkylsebacat und Polydimethylsiloxanen, überzieht, wobei diese Verbindung oder das Gemisch dieser Verbindungen alleine den Überzug bildet oder mindestens 40 Gew.-%,des Gesamtgewichts des Überzugs darstellt, und gegebenenfalls mit einem oder mehreren weiteren Wirkstoffen, sowie mit mindestens einem Zusatzstoff, vorzugsweise mit mindestens einem Füllstoff und mindestens einem Bindemittel, vorzugsweise auch mit mindestens einem Schmiermittel und einem Sprengmittel, wobei der Zusatzstoff bzw. die Zusatzstoffe mit einer stabilisierend wirkenden Verbindung überzogen sein können, intensiv mischt und das erhaltene Gemisch zu einer Tablette verpresst.

14. Verfahren nach Anspruch 13, **dadurch gekennzeichnet, dass** man das erhaltene Gemisch, vorgängig zur Verpressung zu einer Tablette, in Gegenwart von Wasser granuliert, das erhaltene Granulat trocknet und das trockene Granulat vorzugsweise mit mindestens einem Schmiermittel und mit mindestens einem Sprengmittel mischt und zu einer Tablette verpresst.

15. Verfahren nach Anspruch 13, **dadurch gekennzeichnet, dass** man in einem Fliessbettverfahren (i) Candesartan Cilexetil, gegebenenfalls im Gemisch mit einem weiteren Wirkstoff, vorzugsweise Hydrochlorothiazid, oder weiteren Wirkstoffen, zusammen mit mindestens einem Füllmittel und gegebenenfalls einem Sprengmittel, vorzugsweise Lactose Monohydrat und/oder Maisstärke, fluidisiert, und (ii) diesem fluidisierten Gemisch eine stabilisierend wirkende Verbindung, gelöst oder emulgiert in Wasser zusetzt, wobei das anwesende Gemisch mit der stabilisierend wirkenden Verbindung überzogen wird, (iii) das Gemisch gegebenenfalls im Fliessbett mit Bindemittellösung, vorzugsweise Hydroxypropylcellulose, nach bekannter Weise granuliert, das erhaltene Gemisch, als Pulver oder Granulat, trocknet, das trockene Gemisch dem Fliessbett entnimmt, siebt und desagglomeriert, (iv) dem Gemisch ein Sprengmittel sowie ein Schmiermittel zusetzt und nach gleichmässiger Verteilung der Komponenten das erhaltene Gemisch zu Tabletten verpresst.

16. Verfahren nach Anspruch 13, **dadurch gekennzeichnet, dass** man (i) Candesartan Cilexetil, gegebenenfalls im Gemisch mit einem weiteren Wirkstoff, vorzugsweise Hydrochlorothiazid, oder weiteren Wirkstoffen, zusammen mit mindestens einer stabilisierend wirkenden Verbindung in Wasser suspendiert und (ii) mit oder ohne Bindemittel, vorzugsweise Hydroxypropylcellulose, in einem Fliessbettverfahren auf einen oder mehrere Trägerstoffe/- Füllmittel, vorzugsweise Lactose Monohydrat und/oder Maisstärke, aufsprüht, das Gemisch im Fliessbett gegebenenfalls granuliert; anschliessend das erhaltene Gemisch, als Pulver oder als Granulat, trocknet (iii), das trockene Gemisch dem Fliessbett entnimmt, siebt und desagglomeriert, (iv) dem Gemisch ein Sprengmittel sowie ein Schmiermittel zusetzt und nach gleichmässiger Verteilung der Komponenten das erhaltene Gemisch zu Tabletten verpresst.

17. Verfahren nach einem der Ansprüche 13-16, **dadurch gekennzeichnet, dass** man die erhaltene verpresste Mischung mit einem Überzug versieht, vorzugsweise bestehend aus Ethylcellulose, Hydroxypropylmethylcellulose, Hydroxypropylcellulose, Methylcellulose, Carboxymethylcellulose, Hydroxymethylcellulose, Hydroxyethylcellulose, Hydroxypropylmethylphthalat, Celluloseacetat, aus an sich bekannten Polyethylenglycolen, sowie aus Polymeren und Copolymeren von Methacrylsäure.

18. Ausgangsmischung für die Herstellung einer Zusammensetzung gemäss einem der Ansprüche 1-12, **dadurch gekennzeichnet, dass** in dieser Ausgangsmischung der Wirkstoff Candesartan Cilexetil sowie allenfalls anwesende weitere Wirkstoffe in fein verteilter Form vorliegen, vorzugsweise mit einer Korngrössenverteilung, bei welcher 90% der Körner einen mittleren Durchmesser von weniger als 20 Micron (D₉₀<20µm) und vorzugsweise 50% der Körner einen mittleren Durchmesser von weniger als 10 Micron (D₅₀<10µm) aufweisen (aus Anspruch 2) und die Oberfläche mindestens des Wirkstoffs Candesartan Cilexetil mit einem Überzug gemäss Anspruch 1 versehen ist.

19. Verwendung der Zusammensetzung nach einem der Ansprüche 1-12, als Angiotensin II Rezeptor Antagonist, insbesondere zur Behandlung von Bluthochdruck.

## Claims

1. A composition in the form of a tablet or a granulate containing candesartan cilexetil and optionally further active agents and usual additives, **characterized in that** in this composition, the surface of at least the active agent candesartan cilexetil is provided with a coating consisting of a compound or a mixture of compounds selected from tri(C₁-C₆)alkyl citrate, di (C₁-C₆) alkyl phthalate, di (C₁-C₆) alkyl sebacate and polydimethylsiloxanes, wherein this compound or the mixture of these compounds alone forms the coating or accounts for at least 40 wt.% of the total weight of the coating.

2. The composition according to claim 1, **characterized in that** candesartan cilexetil and further active agents that might be present are present in a finely distributed form, preferably with a grain size distribution in which 90% of the grains have an average diameter of less than 20 microns (D₉₀ < 20 µm), and preferably 50% of the grains have an average diameter of less than 10 microns (D₅₀ < 10 µm)

3. The composition according to claim 1 or claim 2, **characterized in that** the compound having a stabilizing effect or the mixture of the compounds having a stabilizing effect are present in a concentration in the range of from 2.0 wt.% to approximately 45 wt.%, preferably in the range of from 5 wt.% to 40 wt.%, based on the weight of the present candesartan cilexetil.

4. The composition according to any one of the claims 1 to 3, **characterized in that** the compound having a stabilizing effect or the mixture of the compounds having a stabilizing effect forms at least 50 wt.%, preferably at least 80 wt.% and preferably at least 90 wt.% of the total weight of the coating.

5. The composition according to any one of the claims 1 to 4, **characterized in that** the compound having a stabilizing effect or the mixture of the compounds having a stabilizing effect is present in a concentration of from 0.2 wt.% to 5.0 wt.%, preferably approximately 0.5 wt.%, based on the weight of the composition.

6. The composition according to any one of the claims 1 to 5, **characterized in that** said composition contains hydrochlorothiazide (CAS no. [58-93-51] or another diuretic as a further active agent, wherein the weight ratio of candesartan cilexetil to hydrochlorothiazide is preferably in the range of from 3:1 to 1:3, preferably in the range of from 2:1 to 1:2, and is in particular approximately 1.3:1 to 1:1.6.

7. The composition according to any one of the claims 1 to 6, **characterized in that** tri(C₁-C₆)alkyl citrate is selected from trimethyl citrate, triethyl citrate, tripropyl citrate, tributyl citrate, preferably from triethyl citrate, tripropyl citrate, tributyl citrate, preferably from triethyl citrate and tributyl citrate, and preferably is triethyl citrate.

8. The composition according to any one of the claims 1 to 6, **characterized in that** di(C₁-C₆)alkyl phthalate is selected from dimethyl phthalate, diethyl phthalate, dipropyl phthalate, dibutyl phthalate, preferably from dimethyl phthalate, diethyl phthalate and dibutyl phthalate, and preferably is dimethyl phthalate and/or diethyl phthalate.

9. The composition according to any one of the claims 1 to 6, **characterized in that** di(C₁-C₆)alkyl sebacate is selected from dimethyl sebacate, diethyl sebacate, dipropyl sebacate, dibutyl sebacate, preferably from dimethyl sebacate, diethyl sebacate, dibutyl sebacate, preferably from diethyl sebacate and dibutyl sebacate, and preferably is dibutyl sebacate.

10. The composition according to any one of the claims 1 to 6, **characterized in that** the polydimethylsiloxane is a liquid compound having a viscosity preferably in the range from 20 - 1300 cSt, and preferably is a linear polydimethylsiloxane having a degree of polymerization (n) in the range of n = 20 - 400.

11. The composition according to any one of the claims 1 to 10, **characterized in that** candesartan cilexetil is present in the composition per tablet in an amount of from 2 mg to 50 mg, preferably in an amount of 2 mg, 4 mg, 8 mg, 10 mg, 16 mg, 24 mg, 32 mg or 45 mg, and the percentage of the active agent is 1 wt.% to approximately 20 wt.% of the total weight of the tablet.

12. The composition according to any one of the claims 1 to 11, **characterized in that** said composition contains additional fillers, binders, lubricants, disintegrating agents, dyestuffs and/or flavoring substances as pharmaceutically usable common additives.

13. A method for producing a tablet according to any one of the claims 1 - 12, **characterized in that** the surface of at least the active agent candesartan cilexetil is coated with a coating consisting of a compound or a mixture of compounds selected from tri (C₁-C₆) alkyl citrate, di (C₁-C₆) alkyl phthalate, di(C₁-C₆)alkyl sebacate and polydimethylsiloxanes, wherein this compound or the mixture of these compounds alone forms the coating or accounts for at least 40 wt.% of the total weight of the coating, and, if necessary, is intensively mixed with one or a plurality of further active agents and with at least one additive, preferably with at least one filler and at least one binder, preferably also with at least one lubricant and one disintegrating agent, wherein the additive or the additives can be coated with a compound having a stabilizing effect, and the mixture obtained is pressed to form a tablet.

14. The method according to claim 13, **characterized in that** the mixture obtained, prior to pressing it into tablet, is granulated in the presence of water, the granulate obtained is dried, and the dry granulate is preferably mixed with at least one lubricant and with at least one disintegrating agent and is pressed to form a tablet.

15. The method according to claim 13, **characterized in that** by using a fluidized bed method, (i) candesartan cilexetil, optionally in a mixture with a further active agent, preferably hydrochlorothiazide, or further active agents, is fluidized together with at least one filler and optionally a disintegrating agent, preferably lactose monohydrate and/or corn starch, and (ii) a compound having a stabilizing effect, dissolved or emulsified in water, is added to said mixture, wherein the mixture present is coated with the compound having the stabilizing effect, (iii) the mixture is optionally granulated in the fluidized bed in a manner known per se with a binder solution, preferably hydroxypropyl cellulose, the mixture obtained is dried as a powder or granulate, the dried mixture is removed from the fluidized bed, sieved and deagglomerated, and (iv) a disintegrating agent and a lubricant is added to the mixture, and after uniformly distributing the components, the mixture obtained is pressed to form tablets.

16. The method according to claim 13, **characterized in that** (i) candesartan cilexetil, optionally in a mixture with a further active agent, preferably hydrochlorothiazide, or further active agents, is suspended in water together with at least one compound having a stabilizing effect, and, (ii) with or without a binder, preferably hydroxypropyl cellulose, is sprayed using a fluidized bed method onto one or a plurality of carrier substances/fillers, preferably lactose monohydrate and/or corn starch, the mixture in the fluidized bed is optionally granulated, the mixture obtained is subsequently dried as a powder or as a granulate, (iii) the dried mixture is removed from the fluidized bed, sieved and deagglomerated, (iv) a disintegrating agent and a lubricant are added to the mixture, and after uniformly distributing the components, the mixture obtained is pressed to form tablets.

17. The method according to any one of the claims 13 to 16, **characterized in that** the pressed mixture obtained is provided with a coating, preferably consisting of ethylcellulose, hydroxypropylmethylcellulose, hydroxypropylcellulose, methylcellulose, carboxymethylcellulose, hydoxymethylcellulose, hydroxyethylcellulose, hydroxypropyl methyl phthalate, cellulose acetate, polyethylene glycols known per se, and polymers and copolymers of methacrylic acid.

18. A starting mixture for producing a composition according to any one of the claims 1 to 12, **characterized in that** the active agent candesartan cilexetil and further active agents that might be present are present in a finely distributed form, preferably with a grain size distribution in which 90% of the grains have an average diameter of less than 20 microns (D₉₀ < 20 µm), and preferably 50% of the grains have an average diameter of less than 10 microns (D₅₀ < 10 µm) (claim 2), and the surface of at least the active agent candesartan cilexetil is provided with a coating according to claim 1.

19. Use of the composition according to any one of the claims 1 to 12 as an angiotensin II receptor antagonists, in particular for the treatment of high blood pressure.

## Revendications

1. Composition se présentant sous la forme d'un comprimé ou d'un granulat, qui contient du candesartan cilexetil et le cas échéant d'autres substances actives, ainsi que des additifs usuels, **caractérisée par le fait que**, dans cette composition, la surface au moins de la substance active candesartan cilexetil est dotée d'un enrobage constitué d'un composé ou d'un mélange de composés choisi parmi le citrate de tri(alkyle en C₁-C₆), le phtalate de di(alkyle en C₁-C₆), le sébaçate de di(alkyle en C₁-C₆) et les polydiméthylsiloxanes, ce composé ou le mélange de ces composés formant à lui seul l'enrobage ou représentant au moins 40 % en poids du poids total de l'enrobage.

2. Composition selon la revendication 1, **caractérisée par le fait que** le candesartan cilexetil et les éventuelles autres substances actives se présentent sous une forme finement divisée, de préférence avec une distribution granulométrique suivant laquelle 90 % des grains ont un diamètre moyen de moins de 20 microns (D₉₀ < 20 µm) et de préférence 50 % des grains ont un diamètre moyen de moins de 10 microns (D₅₀ < 10 µm).

3. Composition selon l'une des revendications 1 ou 2, **caractérisée par le fait que** le composé à action stabilisante ou le mélange de composés à action stabilisante est présent dans une concentration se situant dans la plage de 2,0 % en poids à environ 45 % en poids, de préférence dans la plage de 5 % en poids à 40 % en poids, par rapport au poids du candesartan cilexetil présent.

4. Composition selon l'une des revendications 1 à 3, **caractérisée par le fait que** le composé à action stabilisante ou le mélange de composés à action stabilisante forme au moins 50 % en poids, de préférence au moins 80 % en poids et de préférence au moins 90 % en poids du poids total de l'enrobage.

5. Composition selon l'une des revendications 1 à 4, **caractérisée par le fait que** le composé à action stabilisante ou le mélange de composés à action stabilisante est présent dans une concentration se situant dans la plage de 0,2 % en poids à 5,0 % en poids, de préférence d'environ 0,5 % en poids, par rapport au poids de la composition.

6. Composition selon l'une des revendications 1 à 5, **caractérisée par le fait que** celle-ci contient comme autre substance active de l'hydrochlorothiazide (CAS n° [58-93-5]) ou un autre diurétique, le rapport pondéral du candesartan cilexetil à l'hydrochlorothiazide se situant de préférence dans la plage de 3:1 à 1:3, de préférence dans la plage de 2:1 à 1:2 et en particulier d'environ 1,3:1 à 1:1,6.

7. Composition selon l'une des revendications 1 à 6, **caractérisée par le fait que** le citrate de tri(alkyle en C₁-C₆) est choisi parmi le citrate de triméthyle, le citrate de triéthyle, le citrate de tripropyle, le citrate de tributyle, de préférence parmi le citrate de triéthyle, le citrate de tripropyle, le citrate de tributyle, de préférence parmi le citrate de triéthyle et le citrate de tributyle et est de préférence le citrate de triéthyle.

8. Composition selon l'une des revendications 1 à 6, **caractérisée par le fait que** le phtalate de di(alkyle en C₁-C₆) est choisi parmi le phtalate de diméthyle, le phtalate de diéthyle, le phtalate de dipropyle, le phtalate de dibutyle, de préférence parmi le phtalate de diméthyle, le phtalate de diéthyle et le phtalate de dibutyle et est de préférence le phtalate de diméthyle et/ou le phtalate de diéthyle.

9. Composition selon l'une des revendications 1 à 6, **caractérisée par le fait que** le sébaçate de di(alkyle en C₁-C₆) est choisi parmi le sébaçate de diméthyle, le sébaçate de diéthyle, le sébaçate de dipropyle, le sébaçate de dibutyle, de préférence parmi le sébaçate de diméthyle, le sébaçate de diéthyle, le sébaçate de dibutyle, de préférence parmi le sébaçate de diéthyle et le sébaçate de dibutyle et est de préférence le sébaçate de dibutyle.

10. Composition selon l'une des revendications 1 à 6, **caractérisée par le fait que** le polydiméthylsiloxane est un composé liquide ayant une viscosité se situant de préférence dans la plage de 20 - 1300 cSt et est de préférence un polydiméthylsiloxane linéaire ayant un degré de polymérisation (n) se situant dans la plage de n = 20 - 400.

11. Composition selon l'une des revendications 1 à 10, **caractérisée par le fait que** le candesartan cilexetil est présent dans la composition par comprimé dans une quantité de 2 mg à 50 mg, de préférence dans une quantité de 2 mg, de 4 mg, de 8 mg, de 10 mg, de 16 mg, de 24 mg, de 32 mg ou de 45 mg et la proportion de la substance active dans le poids total du comprimé est de 1 % en poids à environ 20 % en poids.

12. Composition selon l'une des revendications 1 à 11, **caractérisée par le fait que** celle-ci contient comme additifs usuels pharmaceutiquement utilisables, des charges, des liants, des lubrifiants, des désintégrants, des colorants et/ou des arômes.

13. Procédé de fabrication d'un comprimé selon l'une des revendications 1 à 12, **caractérisé par le fait que** l'on enrobe la surface au moins de la substance active candesartan cilexetil par un enrobage constitué d'un composé ou d'un mélange de composés choisi parmi le citrate de tri(alkyle en C₁-C₆), le phtalate de di(alkyle en C₁-C₆), le sébaçate de di (alkyle en C₁-C₆) et les polydiméthylsiloxanes, ce composé ou le mélange de ces composés formant seul l'enrobage ou représentant au moins 40 % en poids du poids total de l'enrobage, et l'on mélange de manière intensive le cas échéant avec une ou plusieurs autres substances actives ainsi qu'avec au moins un additif, de préférence avec au moins une charge et au moins un liant, de préférence également avec au moins un lubrifiant et un désintégrant, le ou les additifs pouvant être enrobés d'un composé à action stabilisante, et l'on presse le mélange obtenu en un comprimé.

14. Procédé selon la revendication 13, **caractérisé par le fait que** l'on granule le mélange obtenu, avant le pressage en un comprimé, en présence d'eau, l'on sèche le granulat obtenu et l'on mélange le granulat sec de préférence avec au moins un lubrifiant et avec au moins un désintégrant puis on le presse en un comprimé.

15. Procédé selon la revendication 13, **caractérisé par le fait que** l'on fluidise, dans un procédé à lit fluidisé, (i) du candesartan cilexetil, le cas échéant en mélange avec une autre substance active, de préférence de l'hydrochlorothiazide, ou d'autres substances actives, conjointement avec au moins une charge et le cas échéant un désintégrant, de préférence du lactose monohydraté et/ou de l'amidon de maïs, et (ii) on ajoute à ce mélange fluidisé un composé à action stabilisante dissous ou émulsifié dans l'eau, le mélange présent étant enrobé par le composé à action stabilisante, (iii) on granule de manière connue le mélange le cas échéant dans le lit fluidisé avec une solution de liant, de préférence de l'hydroxypropylcellulose, on sèche le mélange obtenu, sous forme de poudre ou de granulat, on prélève du lit fluidisé, on tamise et on désagrège le mélange sec, et (iv) on ajoute au mélange un désintégrant ainsi qu'un lubrifiant et, après avoir obtenu une distribution régulière des composants, on presse le mélange obtenu en comprimés.

16. Procédé selon la revendication 13, **caractérisé par le fait que** (i) on met en suspension dans l'eau le candesartan cilexetil, le cas échéant en mélange avec une autre substance active, de préférence de l'hydrochlorothiazide, ou d'autres substances actives, conjointement avec au moins un composé à action stabilisante, et (ii) on projette avec ou sans liant, de préférence de l'hydroxypropylcellulose, dans un procédé à lit fluidisé, sur une ou plusieurs substances supports/charges, de préférence du lactose monohydraté et/ou de l'amidon de maïs, on granule le cas échéant le mélange dans le lit fluidisé ; puis on sèche le mélange obtenu, sous forme de poudre ou de granulat, (iii) on prélève du lit fluidisé, on tamise et on désagrège le mélange sec, (iv) on ajoute au mélange un désintégrant ainsi qu'un lubrifiant et, après avoir obtenu une distribution régulière des composants, on presse le mélange obtenu en comprimés.

17. Procédé selon l'une des revendications 13 à 16, **caractérisé par le fait que** l'on dote le mélange comprimé obtenu d'un enrobage, de préférence constitué d'éthylcellulose, d'hydroxypropylméthylcellulose, d'hydroxypropylcellulose, de méthylcellulose, de carboxyméthylcellulose, d'hydroxyméthylcellulose, d'hydroxyéthylcellulose, d'hydroxypropylméthylphtalate, d'acétate de cellulose, de polyéthylèneglycols connus en soi ainsi que de polymères et de copolymères d'acide méthacrylique.

18. Mélange de départ pour la fabrication d'une composition selon l'une des revendications 1 à 12, **caractérisé par le fait que**, dans ce mélange de départ, la substance active candesartan cilexetil ainsi que le cas échéant d'autres substances actives présentes se présentent sous une forme finement divisée, de préférence avec une distribution granulométrique dans laquelle 90 % des grains ont un diamètre moyen de moins de 20 microns (D₉₀ < 20 µm) et de préférence 50 % des grains ont un diamètre moyen de moins de 10 microns (D₅₀ < 10 µm) (revendication 2) et que la surface au moins de la substance active candesartan cilexetil est dotée d'un enrobage selon la revendication 1.

19. Utilisation de la composition selon l'une des revendications 1 à 12 comme antagoniste du récepteur de l'angiotensine II, en particulier pour le traitement de l'hypertension.
